# EUROPEAN PATENT SPECIFICATION

(11) **EP 1 894 584 B1**
(45) Date of publication and mention of the grant of the patent: **04.08.2010**
(21) Application number: 07121511.5
(22) Date of filing: 26.04.2004
(51) Int. Cl.: A61M 1/00, G01F 1/696

(54) **Flow sensor for a chest drainage device**
Strömungssensor für eine Thoraxdrainagevorrichtung
Capteur de l'écoulement pour un dispositif de drainage thoracique

(30) Priority: 19.06.2003 SE 0301817; 11.09.2003 SE 0302434; 15.09.2003 US 481377 P
(43) Date of publication of application: 05.03.2008
(62) Divisional of application: 04729566.2
(73) Proprietor: Medela Holding AG, 6340 Baar (CH)
(72) Inventor: Charlez, Jarl, 436 42, Askim (SE); Löfgren, Mikael, 431 60, Mölndal (SE); Pettersson, Torbjörn, 413 21, Göteborg (SE)
(74) Representative: Clerc, Natalia

(56) References cited:
- DE-C1- 19 654 014
- US-A- 4 654 029
- US-A- 5 263 380
- US-A- 5 765 432
- US-A1- 2003 010 110

## Description

### Field of the invention

This invention is concerned with an air flow sensor for use in a drainage apparatus for extracting fluids from a body, particularly from the pleural cavity in the human body. Even more specifically it concerns such an apparatus working according to a so called "dry" method, i.e., working without a water-seal and also providing suction pressure control without the need of some kind of water column.

### Background of the invention

Artificial drainage of excessive fluids from the body is necessary for the well being of the patient in variety of medical procedures during and after operations and when the body cannot dispose of such fluids naturally. Fluids to be drained include blood, water and air. A wide range of drainage devices has been developed to drain wounds and larger body cavities. These devices comprise, in general, a chamber for collection of the fluids and an inlet tube for connection between the patient and the chamber. In use, the fluids are drained from the patient via the inlet tube into the collection chamber, which is placed well below the patient to avoid siphoning the fluids back to the patient. Drainage may be caused by gravity, since the chamber is below the patient, or more efficiently, by applying suction to an outlet from the collection chamber to draw the fluid into to chamber.

Various thoracic or cardiac surgical procedures as well as trauma wounds, such as bullet or stab wounds accumulate fluids, such as blood, water or air, in the pleural cavity, between the lungs and surrounding chest wall, this accumulation can interfere with breathing and can cause death by inhibiting the normal pressure changes in the pleural cavity which support normal lung function. In the thoracic cavity, accumulation of fluid such as blood can inhibit healing and patient recovery. In these cases of a persistent leakage of air into the pleural cavity, such as from ruptured bleb - a blister on the lung - the pressure builds up in the cavity until a tension pneumothorax is caused. Then the affected lung; or even both lungs, collapse and the patient can suffocate unless the air is drained from the pleural cavity.

The design of a drainage apparatus for the pleural or thoracic cavities presents special problems. To be effective, the drainage apparatus must remove the fluids promptly and efficiently and prevent their return. It is essential to have one way flow not only to isolate the patient from the atmosphere, so as to avoid infection, but also to prevent fluid, particularly air, from collecting in the pleural cavity and allowing the lung to collapse. The apparatus should also allow relief of over positive pressure caused, for example, by the patient coughing or fluid displacement in the collection chamber when used for gravity drainage and also allow relief of over-negative pressure caused by the application of too much suction to the chamber.

The source of suction, when used can fail and therefore the apparatus should be constructed to function as gravity drainage apparatus as a precaution against this eventuality so that fluid can still be drained from the patient. Display of an accurate suction level reading is also needed so that the physician can readily monitor or easily re-adjust the level of suction during the course of treatment to the patient. Moreover when there is substantial quantity of air leaking from the lungs into the pleural cavity, this air must be withdrawn by the apparatus, which should be constructed to accommodate such air leaks and sudden changes in the rate of leakage while indicating the leak to the users.

The prior art typically uses a water seal in the drainage apparatus between the collection chamber and the source of suction, or between the chamber and the atmosphere in the case of gravity drainage, to isolate the patient from the atmosphere and keep air from getting back into the chest. The water seal also serves as an indicator of air leakage from the patient when air bubbles through the water, the amount of bubbling serving as a guide to the physician of the degree of air leakage. Also motion of the water level indicates the patency and sampling of the patient. Unfortunately, such seals have not been reliable in preventing contamination of the patient and have been inconvenient and unreliable in use, as described below. The simplest form of such a drainage apparatus comprises a single bottle partially filled with water and serving as a water seal chamber. The bottle has an inlet tube from the patient extending below the water and on outlet tube communicating with the air space above the water. This is a rudimentary arrangement, which provides no control over, or indication of, the pressure in the collection chamber. As the drained material gradually fills the bottle, the resistance to drainage increases.

To improve on this design, a plurality of bottles has been used, connected in series between the patient and a source of suction. Normally three bottles are used: the first, connected to the patient as a trap, collects the fluids; the second, partially filled with water, forms the seal; and a the third acts as a pressure regulator by having a tube open to the atmosphere and extending below water in the bottle. The dept of this tube below the water determines the maximum amount of suction that can be drawn through the system because once sufficient suction is reached to drain air down the tube from the atmosphere and bubble through the water, increasing the level of suction only increases the rate of bubbling and consequently the level of suction in the apparatus is limited. However, the separate bottle arrangement is cumbersome and awkward to use and is very difficult to clean for re-use. This has lead to design of a variety unitary, sterilizable and disposable drainage apparatus. U.S. Patents No. 3,363,626, 3,363,827, 3559,647, 3,683,913 and 3,853,128 show the development of rigid, disposable, plastic drainage apparatus, which has adapted the three-bottle arrangement into a unitary structure. A drainage apparatus which includes a water seal, requires a relatively long time to set up because the apparatus must be primed with a certain volume of sterile water to generate the and to fill the U shaped manometer chamber. This volume of water is relatively large and usually more than one chamber has to be filled. U.S. 3,363,627 attempted to overcome this problem by making the connections between the chambers external to the apparatus and above the chambers so that the apparatus could be supplied pre-filled. However the water seal systems suffers from a number of disadvantages, such as evaporation, nuisance, overfill and contamination which all can effect the safety aspects of one-way-valve mechanisms. Lately there have been modifications done by companies like Genzyme (Deknatel) and Atrium and they have overcome the above-described problems of water seal aspects by launching a dry one-way-valve mechanism.

The depth of water in the pressure regulator chamber, as mentioned above limits the amount of suction that can be applied to a water seal system. The maximum suction that can be applied to commercial water seal reservoirs are about 30 cm water, since a much longer pressure control chamber is very cumbersome and expensive to manufacture. This is inadequate since suction up to about 60 cm water is sometimes required. Further, a water seal drainage apparatus is normally relatively heavy and large which is a disadvantage both for hospital staff and for the patient who often holds the apparatus when he is moved. Another serious drawback is the relatively complex instructions required for; it's use, which can lead to mistakes and wasted time. In attempts to overcome these deficiencies, a few waterless devices have been developed. U.S. Patent No. 3,830,238 discloses a simple arrangement which is a waterless, one-chamber device containing an inlet tube having a one-way-valve to prevent flow of fluid back to the patient and a bellows arrangement coarsely measure the pressure in the pleural cavity. But this device does not control or accommodate pressure fluctuations or indicate the actual pressure in the chamber.

Davol, Inc. has also produced a device without a water seal under the trademark Thoraklex and so has Atrium and genzyme (Deknatel). The outlet of these three products and trademarks (Deknatel, Atrium and Thoraklex) is connected to a negative pressure control consisting of a screw ore a button for adjustable occluding the outlet passage to regulate the degree of suction. The outlet also includes a float ball gauge calibrated to indicate the suction applied to the patient and connected the atmosphere and the outlet. However the real pressure in the pleural cavity is not visualized in these systems, but to overcome that to high negative pressure are build up, these apparatus incorporates an over-negative-pressure relief valve, which vents to atmosphere at a pre-set negative pressure to prevent the vacuum reaching too high a level (60 cm water). This valve is set in a passage between the atmosphere and the collection chamber and comprises a one-way spring-loaded valve. Since actuation of the valve allows air from the atmosphere into the collection chamber, which is open to the patient, it is necessary to provide a filter in the passage. This arrangement suffers from disadvantages that the suction level can build up rapidly in the collection chamber while the valve is opening and although the filter is intended to prevent bacteria entering the chamber, placement of the valve opening directly to the chamber still exposes the patient to risk of infection and also increases manufacturing costs of the apparatus.

US 5,765,432 discloses a hot wire flow sensor comprising a bridge circuit and a heating resistor.

US 4,654,029 discloses a system for electronically monitoring and controlling the drainage of fluids from a body cavity, including transducers for measuring suction air flow, patient air flow, patient negativity and the like, and displays for rendering measured values in legible form.

US 4,889,531 discloses a pressure regulator including high and low pressure chambers separated by a divider having an opening, a closing member biased to a closing position for closing the opening with a biasing force according to a desired pressure differential between the chambers.

WO 01/98735 discloses a hot wire flow rate sensor having a bridge circuit with a sensing resistor which avoids saturation of the signal processing circuitry at high gas flows rates by increasing a bias signal to a differential amplifier in the signal processing circuitry in a step-like manner as the flow rate responsive signal to the differential amplifier from the sensing resistor increases.

GB 2 077 397 discloses a vacuum regulator comprising a body having parallel passages and a cap attached by a screw thread over one end of the body defining with the body a chamber containing a diaphragm arranged to co-operate with a seating at the end of a passage.

FR 2 560 770 discloses a vacuum regulator similar to the one in GB 2 077 397.

US 4,710,165 discloses a wearable, variable rate suction/collection device.

Therefore, there exists a need for a drainage apparatus, which avoids the disadvantages of the water seal, water U-shaped manometers, pre-set suction levels and lack of visualization of healing control Further, a such apparatus must be able to function under conditions of gravity drainage or vacuum drainage and, in the latter case, apply stable and acceptable levels of suction to the patient. There is also a need for a simple, reliable sensor in the drainage apparatus, to indicate accurately the pressure conditions in the collection chamber over a range of negative pressures. Furthermore there is also vital to measure quantity of air that is evacuated over time, which in it self indicates the progress of healing.

In addition, it's desirable that the apparatus is not to expensive to manufacture and that it could be constructed to reduce the need for guessing the intra pleural pressures and volume of air leakage, and that it will be convenient for the patient and medical staff to use. Many forms of drainage apparatus currently available are not only insufficient to monitor due to their pre-set pressure levels and automatic relief valve, opening first at a negative pressure exceeding 60 cm water. The lack of suitable surveillance means, increased use of X-ray examinations and hasn't really improved the management of treatment with drainage apparatus in aspects of making healing processes more objective or visualized.

A future scenario for standard of care (SOC) should include the possibility to collect and transfer data for patient documentation. This is done in most other surveillance applications in the medical field today.

### Summary of the invention

An objective of the present invention is to provide an apparatus, which overcomes the disadvantages of prior art and which is reliable and convenient to use under a variety of operating conditions and locations. It is thus an objective to provide an apparatus for draining fluid from a patients pleural cavity while maintaining an amount of suction pressure in said cavity where said apparatus do not need priming with a certain amount of water. It is a further objective to measure and display important treatment parameters such as present interpleural or pericardial pressure, present air flow, and accumulated amount of drained liquid. Further objectives include also to provide an apparatus that is resistant to position changes and dropping, and that will function without any external electrical power source or batteries. The apparatus should also include safety means to eliminate the risk of too high suction pressure and the risk of causing a non-suction pressure. The apparatus is also preferably provided with means for transferring the above mentioned treatment parameters to an external electronic device e.g. a personal computer (PC) or a personal digital assistant (PDA)

The present invention is defined in claim 1.

Thus, an embodiment of the invention comprises a flowmeter, which comprises a sensor by means of a thermistor, which is mounted in an air channel for measuring the volume of air passing through the system over time. The channel has an inlet for directing the flow of gases from the collection chamber, and an outlet for letting the airflow from the apparatus. The sensor comprises a thermistor sensor, which is first heated up and then cooled off by the airflow in the channel. Heat differentials cause the sensor in use to be electrically polarized. Four thermal resistors of the same nominal resistance is arranged in a row in said channel along a direction parallel to the air flow and with a heating resistance arranged between resistor number two and three. Said resistors are electrically coupled in a Wheatstone-like bridge such that the voltages of two voltage dividers are compared i.e., the voltage over resistor number four is compared with the voltage over resistor number 2 and where one current is flowing through resistors one and four, and another current is flowing through resistors three and four. The so formed differential voltage is measured under two conditions, i.e., heated with the heating resistor and unheated. The difference between the voltages obtained under heated and unheated conditions are subtracted from each other and the resulting difference is used as representative of the value of the air flow rate.

### BRIEF DESCRIPTION OF THE DRAWINGS

The invention is described with reference to the accompanying drawings, in which:
Figure 1 is an explanatory sketch showing the working principle of the present invention.
Figure 2a shows a suction pressure regulator, partly in cross section, for a drainage device.
Figure 2b shows, in cross section, a suction pressure regulator.
Figure 3 shows a an overview of an embodiment of an apparatus for use with the present invention
Figure 4 shows a collection chamber, with the lid removed for clarity, for a drainage device.
Figure 5a shows, in cross section, a non return valve unit.
Figure 5b shows, partly in cross section, a detail of the non return valve unit in fig 5a.
Figure 6 shows a block diagram of a measuring and display unit for use in a drainage device.
Fig. 7a shows a photograph of a MEMS flow sensor according to an embodiment of the invention.
Fig. 7b shows an enlargement view of a part of the sensor in fig. 7a shoving four serpentine like sensor resistors
Fig. 7c shows a bridge circuit of a preferred way of coupling the sensor resistors.
Fig . 8a shows a circuit diagram of a sensor circuit board for an embodiment of the present invention
Fig. 8b is a circuit diagram showing miscellaneous components of the sensor circuit board
Fig. 9a shows a circuit diagram of a controller circuit board for a drainage device.
Fig. 9b.shows a connectors overview.
Fig. 10 shows a flow chart of a method of obtaining a value representative of an air flow for use with one embodiment of the invention.

### DETAILED DESCRIPTION

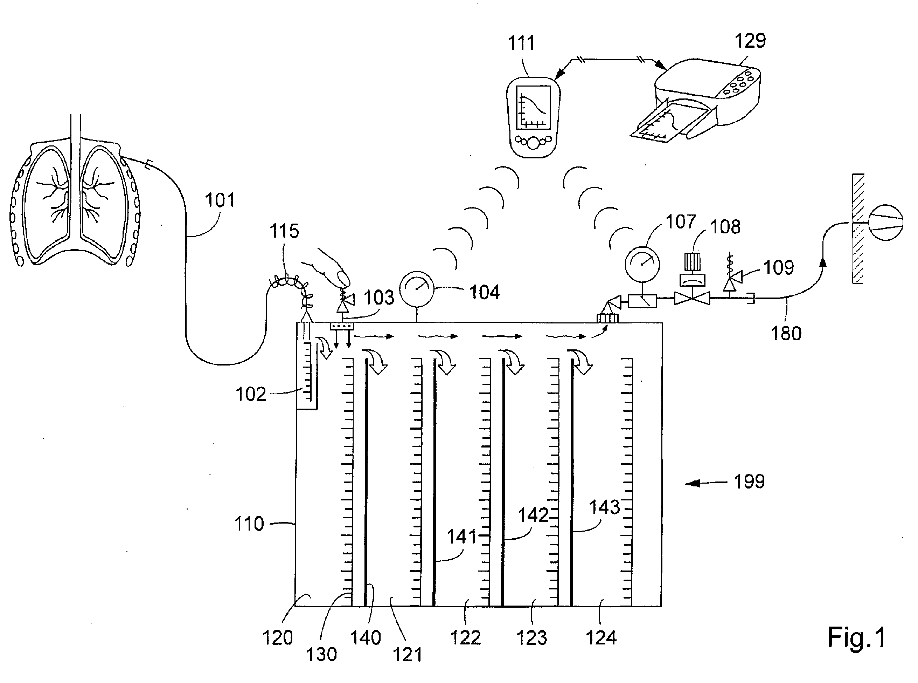

Figure 1 is an explanatory sketch showing the working principle of a drainage apparatus 199 for use with the present invention. The figure shows a drainage tube 101, preferably of a kink resistant type, having a first and a second end. Said first end is intended to be connected to a patients body to drain fluid from typically a pleural or a pericardial cavity. Said second end is connected to a drainage apparatus 199 having an airtight collection chamber 110. The drainage tube 101 is provided with a reinforced part 105 near its second end close to an inlet opening of the collection chamber 110. Fluids that are extracted from the body include blood, pus and protein containing fluids with a high water content. The collection chamber is intended to be used in an upright position. Fluids entering the collection chamber 110 do, owing to gravity, first enter a first compartment 102, a paediatrics measuring chamber 102 having defining walls 113,114. Said chamber 102 is provided to enable a physician, a nurse or other health care personnel to make an optical reading on a first scale 112. The material of the collection chamber 110 is of an optically transparent type, e.g polycarbonate plastic or the like, allowing for easy and convenient readout of the fluid level inside the collection chamber 110. The paediatrics measuring chamber 102 has a relatively small transverse cross section so that when fluid are filling the chamber the level is increasing relatively much for a certain amount of fluid, making it easy to detect small variations in the fluid level. This is of course of most use when treating small children, whose bodies, heart and lungs are small, and therefore also the amount of fluid that will be drained is small. Alternatively, the paediatrics measuring chamber 102 is having its own set of defining walls making assembly easy by allowing for easy sliding the paediatrics chamber 102 into place in e.g. a dovetail slot.

When treating an adult patient producing a large amount of excess fluid in his pleural or pericardiac cavity, fluid will fill the paediatrics measuring chamber 102 and enter a second compartment 120, having a corresponding measuring scale 130. As fluid levels increase further the second compartment becomes full and the fluid flows over a first dividing wall 140 to a third compartment 121. Preferably the collection chamber comprises a number of such compartments 121-124 divided by dividing walls 140-143, preferably five to six compartments, allowing for easy readout of up to for example 2500 millilitres of fluid.

The collection chamber is provided with a vacuum reducer valve 103. The collection chamber is also provided with a pressure measuring means 104.

Air evacuated from the patient passes over the dividing walls 140-143 and is sucked away via an outlet of the collection chamber 110, via protective means 105, comprising a capillary breaker 105, further via a non return valve 106, still further via a flow measuring means 107, a suction regulator 108, a high pressure valve 109, and finally via a suction supply tube 180 to an external source of suction pressure.

The pressure measuring means 104 and/or the flow measuring means 107 may be of a type connectable to an electronic measuring unit capable of wireless communication, e.g. infrared communication, with a computer 111 for further processing of information. A printer 129 can be connected to the computer 111 for the printing of parameters, trend diagrams on the like. The apparatus can also be provided with display means, connected to the electronic measuring unit, for displaying actual parameter values, such as collection chamber pressure, and current flow.

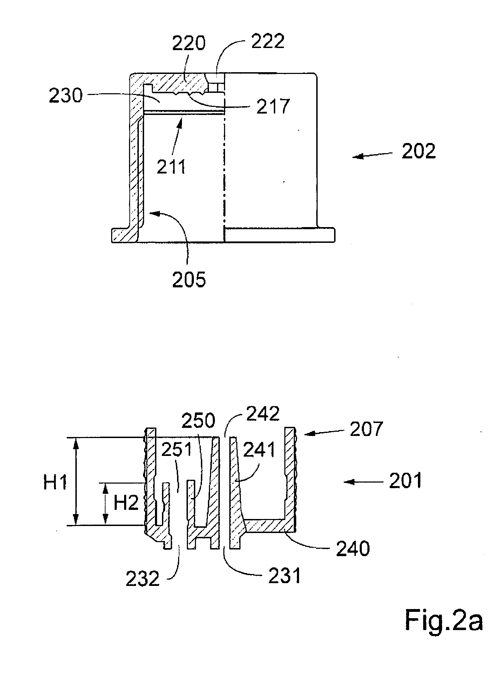

Figure 2a is a view of a suction pressure regulator, partly in cross section, for a drainage device. The regulator preferably comprises two main parts, a house 201, and a turnable cap 202. The cap 202 has preferably a cylindrical shape so it can be screwed onto the house 201. For this purpose the cap is provided on its inner cylindrical surface with threads 205, and the house is correspondingly on its outer cylindrical surface provided with corresponding threads. The threads are airtight in themselves or, in another embodiment, means such as O-ring are provided to provide an airtight connection between the house 201 and the cap 202. The cap 202 is provided with a diaphragm 211 attached inside the cap 202 near an inner surface 217 of a top part 220. The inner surface 217 is preferably provided with a rough surface or pimple-like protrusions preventing the diaphragm 211 from adhering to the top inner surface 217. The diaphragm 211 is attached to the inner cylindrical walls in an air tight manner. The top part 220 is provided with a hole 222 for letting air of atmospheric pressure to enter and leave a small space 230 above the diaphragm 211.

The house 201 comprises connections 231, 232 for supply suction pressure, and regulated suction pressure respectively. The supply connection 231 has a fluid connection through a bottom plate240 of the house 201 to a prolonged nozzle 241 inside the house 201. Said nozzle part having a top orifice 242 facing the diaphragm 211 so that an airtight contact can be achieved between the diaphragm 211 and the orifice 242. The nozzle part is preferably arranged in the centre of the bottom plate 240. Also the regulated pressure connection 232 preferably comprises a prolonged nozzle part 250 inside the house 201, having an orifice 251.

The regulator functions as described below. In some point in the regulatory process the diaphragm is in air tight close contact to the orifice 242 of the supply nozzle 241. If the pressure inside the house 201 raises, i.e., the suction pressure becomes less, the diaphragm 211 will bend away from the supply orifice 242, thereby open for a supply suction pressure supplied via the nozzle 241, causing the pressure to fall. As the pressure falls, the diaphragm will bend towards the supply orifice 242 again and eventually make close contact to said orifice 242, thereby closing off suction pressure supply. This process is repeated and the pressure is regulated.

To adjust the pressure the cap 202 is turned clockwise or anticlockwise like a knob so that a distance between the diaphragm 211 and the supply pressure nozzle orifice 242 becomes less or more.

Another feature of the regulator is the feature of handling the problem of temporary formation of condensation water. Air coming from the human body normally contains 100% humidity at 37 degrees Celsius. As the temperature falls this humidity will fall out as water. To lower the risk of influence of such water in the regulator the hight H2 of the regulated pressure prolonged nozzle 250 is approximately half the hight H1 of the supply pressure nozzle 241. Water formed inside the regulator house 201 will stay at the bottom of the house, or leaving towards a collection chamber via the regulated pressure inlet 232, without affecting the diaphragm.

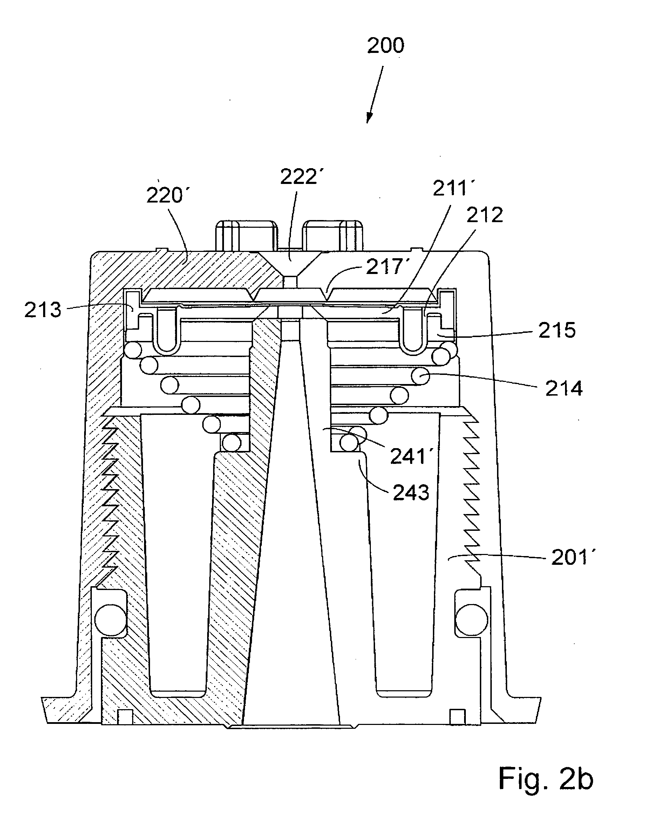

Figure 2b shows, in cross section, a pressure regulator. Those parts having a corresponding part in fig. 2a have the corresponding reference numerals with the addition of "prim" ('). The regulator in fig. 2b is based on the same principle as in fig.2a i.e., the pressure is adjusted by moving a membrane (211, 211') closer to or further away from a suction pressure supply nozzle (241, 241'). The regulator 200 comprises a regulator house 201' and an adjustable cap 202', where said cap is provided with a membrane 211'arranged close to its closed end 220'. The membrane is provided with a bellow 212 allowing the membrane to move in a downward-upward direction following the orifice of the prolonged nozzle 241' when the cap 202' is turned, and the pressure is adjusted is described above. The membrane 211' is also provided with a rim 213 providing structural stability an a means for being able to be kept on place. The membrane 211' is clamped between the upper part 220' of the cap 202' and a slide ring 215, which is kept in position by means of a conical spring 214.

Said conical spring 214 serves the double purpose of on the one hand to provide means of keeping the membrane 211' in position, and on the other hand to provide means for keeping the cap 202' and the house 201' in a firm relationship avoiding loose or play between said cap 202' and house 201' that may otherwise adversely affect the pressure regulatory functioning. The conical spring has a large and a small end, the large end abuts the slide ring, while the small end abuts a shoulder 243 on the prolonged nozzle 241'.
The spring 214 is designed to function in the following way: starting with an unloaded spring; when the axial load increases, the turns having the greatest radii is the most active. Said turns undergo deformations successively, one turn after the other, and the smaller turns become more active. By using a conical spring instead of an ordinary spring with constant diameter, wear in threads of the regulator is decreased and jarring is avoided. A wire diameter of 1.4 millimetres has proved to be appropriate. The conical shape allows for low build and do not expand outwards which prevents the origin of sounds when adjusting the suction pressure. Due to the progressive spring forces, a more distinct and controlled fine adjustment of the pressure at low flows is achieved.

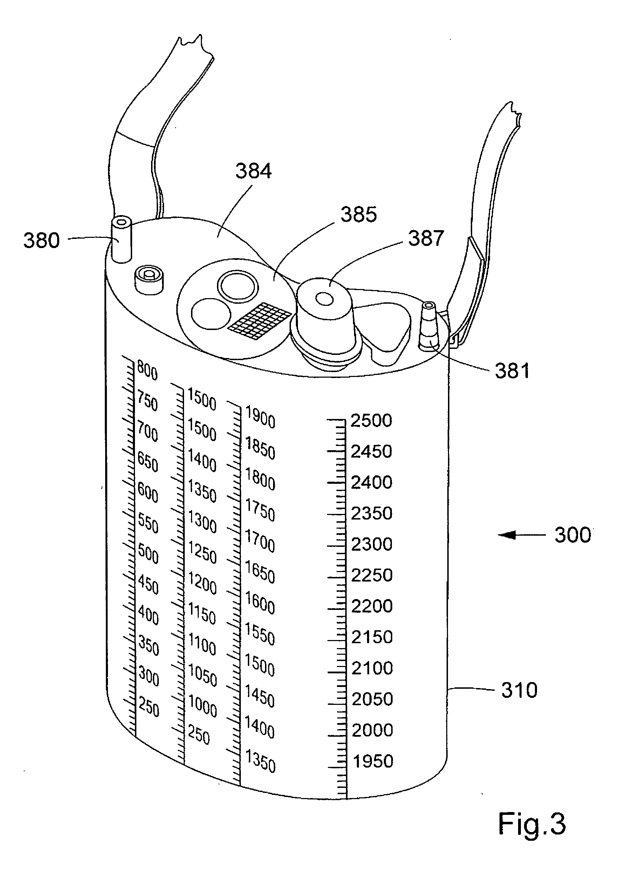

Figure 3 shows an overview of a drainage apparatus 300 for use with the present invention. A collection chamber 310 is provided with an airtight lid 384 having an inlet connection 380 connectable to a drainage tube from the patient, and an suction pressure supply connection 381. The suction pressure supply connection 381 is connected via internal channels in the lid 384 to a suction pressure regulator 387. Regulated suction pressure is connected to the internal of the collection chamber 310 via a non-return valve and a capillary breaker (not shown in fig. 3) A electronics unit 385 is provided in the lid having a pressure display, a air flow display and one or more solar cells for supplying all the power needed to power the electronics unit 385. The unit 385 is made easy removable allowing environmentally correct disposal of different kind of waste when the apparatus is to be disposed.

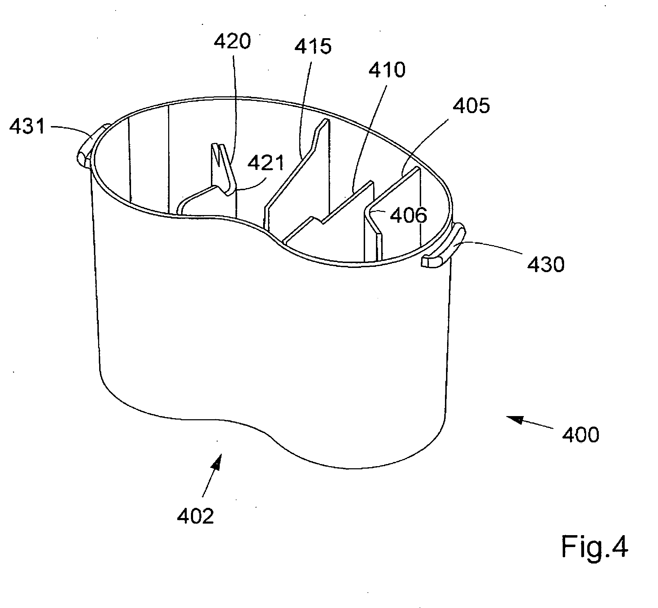

Figure 4 shows a collection chamber 400, with a lid removed for clarity, for a drainage device. The chamber 400 has a cross section providing an indentation running from a bottom part 402, not visible, to the lid, preferably giving the chamber 400 a kidney shaped cross section adding to the stability and rigidness of the chamber 400. Said chamber 400 is divided into compartments by a number of separating walls 405,410,415, 420.. The separating walls are provided with fluid passing cuts near the lid to allow fluid to flow from a full compartment to a not yet full compartment. These separating walls and cuts are arranged in a labyrinth manner preventing or reducing the risk of fluids from entering further compartments if the chamber accidentally falls on the side or otherwise is tilted in a non upright position. Two nearby separating walls have their fluid passing cuts formed at opposing sides i.e. near the indented side or near the non indented side. Some of the walls are also provided with cuts to give room for the lid and the electronics in the lid the two outermost dividing walls 405, 420 are provided with longitudinal running bends 406, 421 to further add to rigidness and stability. The lid is air tightly sealed to the collection chamber by means of silicone seal, plastic welding or other suitable method. The chamber 400 is provided with fastening means for a strap with VELCRO adjustment means or the like for easy suspension and carrying of the drainage device.

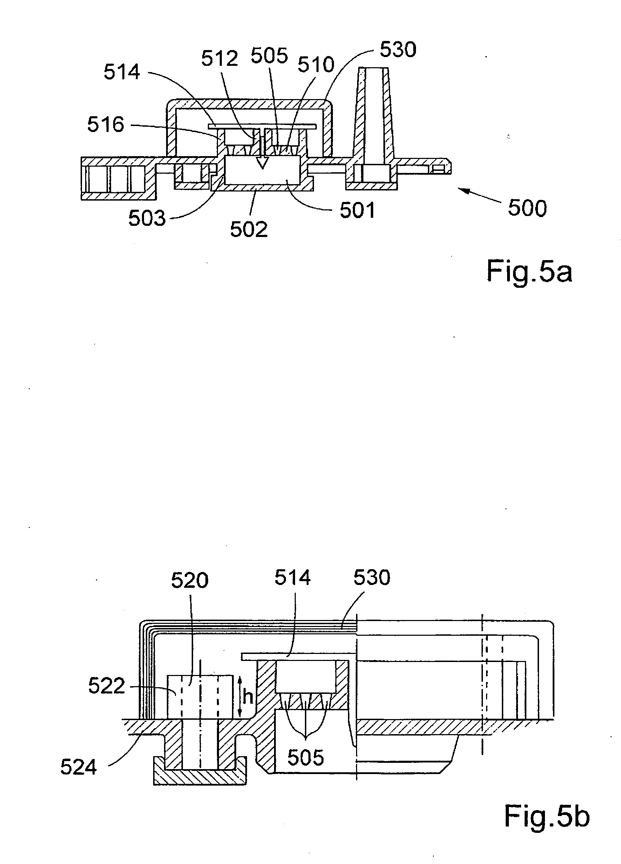

Figure 5a and 5b shows, in cross section, a non return valve unit. The evacuated air enters an atrium 501 having a floor 502 and vertical walls 503. The atrium 501 is upwardly defined by a capillary breaker 510 in the form of a horizontal wall 510 having a number of conical holes 505 in it. The holes 505 have a smaller and a wider end, and the smaller end is directed downwards in fig. 5a and 5b such that air streaming through the atrium 501 and the capillary breaker 510 will have a less water content after the breaker than before said breaker 510. The wall 503 is preferably continued upwards and a central column 512 is formed that holds a horizontal membrane 514 preferably of rubber of another flexible material. A non return valve is formed as the membrane forms an air tight seal with an upper part 516 of the vertical wall 503, as long as the pressure is lower on the side of the membrane 514 that is facing the capillary breaker than on the other side. The evacuated air then enters a measuring channel inlet 520 having vertical wall 522 of a height h that together with an airtight cover 530 forms a condense trap 524 further reducing the possible water content of the evacuated air.

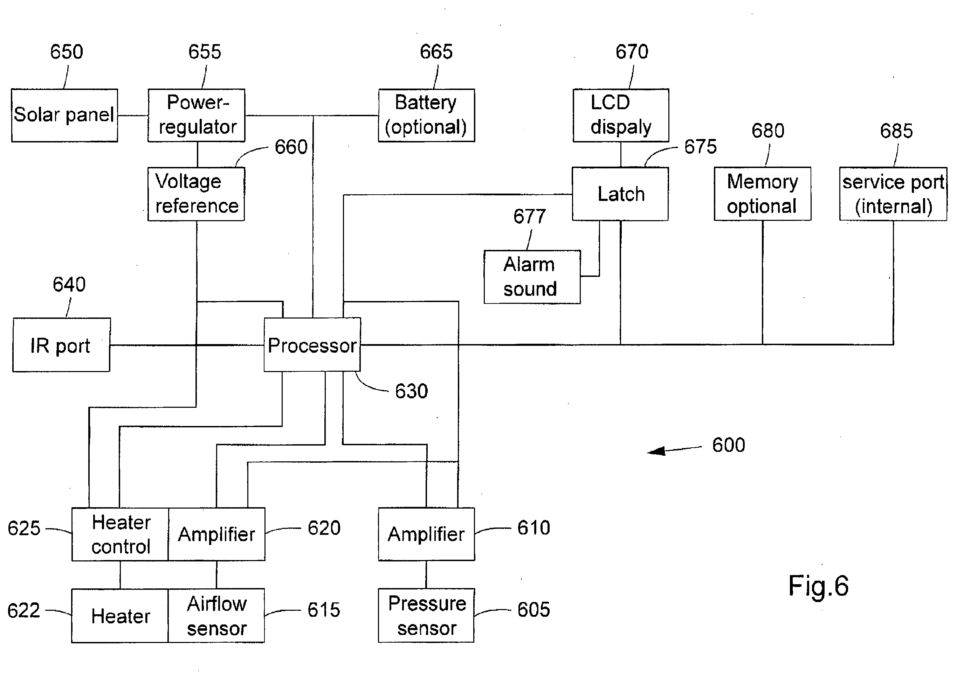

Figure 6 shows a block diagram of a measuring and display organ 600 for use in a drainage device. Said measuring and display organ 600 include a pressure sensor 605 for measuring the pressure in a pressure measuring part of the drainage device that is in pressure connection with the body cavity. Said measuring and display organ 600 also include an airflow sensor 615 for measuring the airflow in an airflow measuring part of the drainage device, representative of the airflow from said body cavity. The sensors 605, 615 are connected to a processor 630 and send signals representative of said pressure and flow to said processor 630. Said processor processes the signals from the sensors 605, 615. Such processing may include analogue to digital conversion of signal values, compensations for nonlinearities etc. The processor 630 also transforms pressure and flow values to signals suitable to drive a display unit 670. The processor is connected to a display unit 670. Said display unit 670 receives signals from the processor 630 representing the pressure and flow values. The display unit 670 displays said values preferably in both a digital and in an analogue format making the values clearly readable for medical personnel handling the nursing and treatment of the patient, making parameters of progress as well as deterioration in the recovery process easily quantifiable. Preferably, a button is arranged, so that when pressed, the cumulative airflow, representative of the last hours cumulative air leakage is shown on the display 670.

The processor 630 is preferably of the flash type, i.e., it has an internal flash memory which contributes to low power consumption. The measuring and display organ 600 is powered from a solar panel 650 provided with a power regulator 655. The organ 600 may be powered from a battery 665. The processor 630 is preferably connected to an IR port 640 for transferring information to an external device such as a personal computer or personal digital assistant. The data transferring may be accomplished by wireless BLUETOOTH-means.

The display unit 670 is preferably of a liquid crystal display (LCD) type or another low power consumption type and may be connected to the processor over a latch 675. The pressure value is preferable displayed as a two-digit number representative of the pressure in centimetres water column. The air flow is preferably displayed as a two digit number expressed in litres per minute.

### Pressure sensor

The pressure sensor 605 is preferably of a micro electromechanical system (MEMS) type. A suitable sensor is for example the MS761D from Intersema Sensoric SA, BEVAIX, SWITZERLAND (http://www.intersema.ch). In a preferred embodiment the pressure sensor 605 include a small rectangular area provided with a measuring resistance along each of the four sides. Electrical connections may be provided at all four corners. The pressure sensor is provided with means to withstand liquids such as water with ions, e.g. salt.

### Pressure alarm

The measuring and display organ 600 may comprise pressure-alarm means. Said alarm-means comprises means for setting user-defined, low and high alarm limits. Said alarm-means also comprises a sound-unit 677 or other audio or visual means to indicate that a limit is reached. The processor 630 is provided with the necessary functionality to compare set limits with actual pressure values and to initiate proper indication if a limit is reached. Said functionality is preferably implemented as computer program code in the processor 630.

### Air flow sensor

In a preferred embodiment the air flow sensor 615 include a micro-electro-mechanical system (MEMS) sensor chip of approximate size 5x5mm or less. The sensor includes a heater element with 2 measuring resistances on each side of it, and a membrane upon which the heater element and other resistors are applied. Said membrane has a size of roughly 125x 250 um. Tables show that the power requirement for keeping the heater element at 200 degrees Celcius should be in the 10mW magnitude. The heater element includes a conductor or "wire" of a size of approximately 3um diameter (cross section) x 100um length. It is calculated that it will be necessary to drive 10mW into the heater element from a 2 Volt voltage source, equivalent to a solar cell under load, and therefore the conductor should have a resistance of roughly 400 ohms. The chosen heater is approximately 24um long and have a diameter or cross section of 6um. A preferred resistance for the measuring resistances will be 2k-20k because of the desire to keep power consumption together with heating down. In one embodiment said measuring resistances comprise 4 identical 3um diameter 100um long serpentines over said membrane. Two extra temperature reference resistors may be designed in as well to increase accuracy. The expected signal at full airflow (specifically at zero flow, because the signal will be inverse proportional to the flow) will be 0,5-2%. The output from the sensor is preferably resistance. At zero airflow the processor will recognize a nominal electrical resistance. At high airflows the processor will recognize an electrical resistance differing from the nominal with typically 2 percent. The sensor provides preferably a real resolution of 10bits within a measuring range of 5ml - 51 per minute, the least detectable signal being 2ppm with a margin factor of 4. The measuring response is preferably logarithmic giving a sensor more sensitive at low air flows.

The heater wire is designed to withstand high temperatures, but relatively low powers can still break the device. Therefore driver electronics means is provided that drive the heater within acceptable power and temperature. To reduce power consumption the heating is pulsed, i.e., current is applied to the heater element in pulses.

The air flow sensor 615 is preferably arranged in a channel of approximately 5 millimeters cross section that is provided with a narrowing giving rise to a narrow channel of approximately 2 quadratic millimeters in cross section where the airflow sensor 615 is provided. Because of this narrowing the flow speed will become higher making it easier to measure small flows. The sensor is preferably arranged at the uppermost portion of said narrow channel making it less sensitive to condensed water.

The resistors is in a preferred embodiment made of polycrystalline silicon (poly-Si) and conductors on the sensor board is made of metal A1 (Cu/Si). A thermal oxide is arranged between said resistors and conductors and a silicon substrate of approximately 380 microns of thickness.

### Air flow sensor circuitry

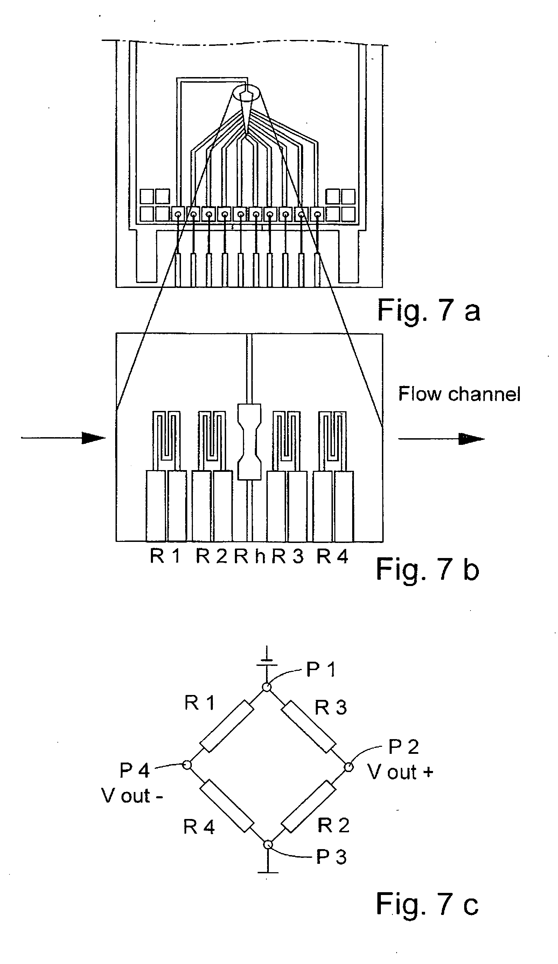

Fig. 7a shows a photograph of a MEMS flow sensor according to an embodiment of the invention. Fig. 7b shows an enlargement view of a part of fig. 7a shoving four serpentine like sensor resistors denoted first R1, second R2, third R3 and fourth R4 resistors and a heating resistor Rh. In the fig. 7b a reference direction Ref is shown as arrows pointing from left to right. In the assembled drainage apparatus this direction is preferably the same as the direction of the air flow to be measured. The resistors R1-R4 is arranged from left to right, R2 to the right of R1, R3 to the right of R2 and so on. The heating resistor Rh is placed between R2 and R3, such that the distance from the heating resistance to the second resistance R2 is the same as the distance from the heating resistance Rh to the third resistance R3.

Fig. 7c shows a bridge circuit of a preferred way of coupling the sensor resistors R1-R4. The bridge is defined by four imaginary connection points denoted a first P1, a second P2, a third P3 and a fourth P4 connection point. The first sensor resistance R1 is connected between the first P1 and the fourth P4 connection points. The second sensor resistance R2 is connected between the second P2 and the third P3 connection points. The third sensor resistance R3 is connected between the first P1 and the second P2 connection points. The fourth sensor resistance R4 is connected between the third P3 and the fourth P4 connection points.

The described way of connecting the four sensor resistances in a bridge has been shown by simulation to give better characteristics compared to using only the second R2 and third R3 sensor resistors together with a pair of dummy resistors in the bridge, and similarly, has also been shown to give better characteristics compared to using only the first R1 and fourth R4 sensor resistors together with a pair of dummy resistors in the bridge. Here, a dummy resistor is a resistor not being exposed to flow of air.

### Boards and circuitry

Fig . 8a shows a circuit diagram of a sensor circuit board for an embodiment of the present invention. The J2 in the upper left corner connects the sensor circuit board to a small chip comprising the air flow sensor of fig. 7a. A voltage measured over the second P2 and fourth connection points is fed to an amplifier circuit comprising operational amplifiers U101-A and U101-B. The resulting signal is then fed to a flow offset compensation circuit comprising an operational amplifier U102-A making it possible to adjust for individual sensor differences. An adjusted flow signal output from said offset compensation circuit is fed to the microcontroller U101 which can be a ATMEGA48V. Further, a pressure sensor S101, which can be a MS761D is connected to an amplifier circuit comprising operational amplifiers U101-C and U101-D. The resulting signal is then fed to a pressure offset compensation circuit comprising an operational amplifier U102-C making it possible to adjust for individual sensor differences. The current feeding the pressure sensor S101 flows through the resistor R133. A voltage over the resistor R133 is fed to a conditioning circuit comprising a operational amplifier U 1 02-D. The voltage from said conditioning circuit is then fed to the microcontroller U101. Said voltage from said conditioning circuit is representative of the temperature of the pressure sensor S101 and can be used by the microcontroller U101 to compensate for temperature variations.

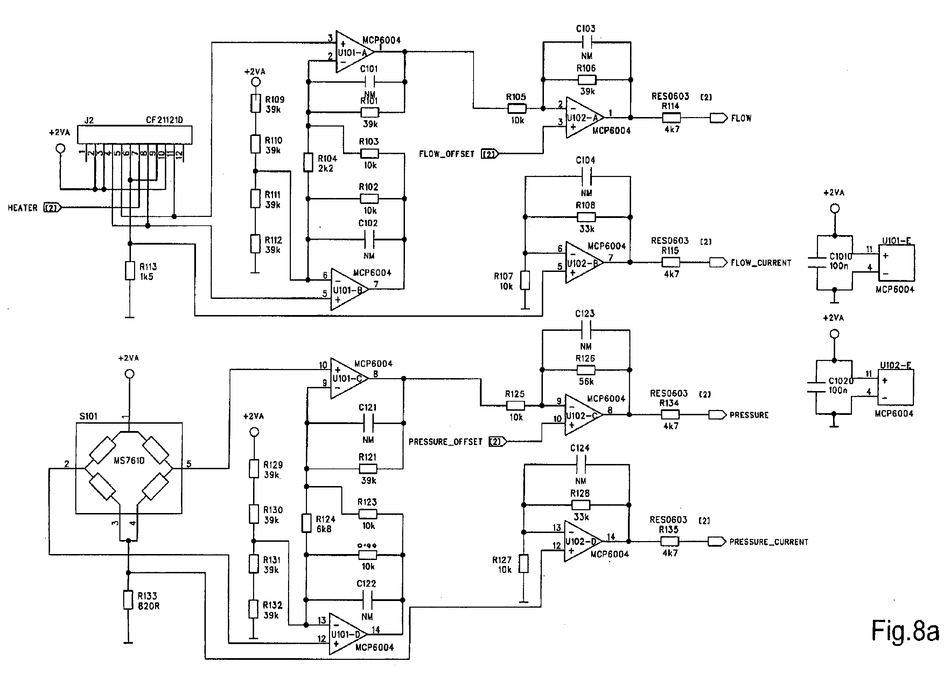

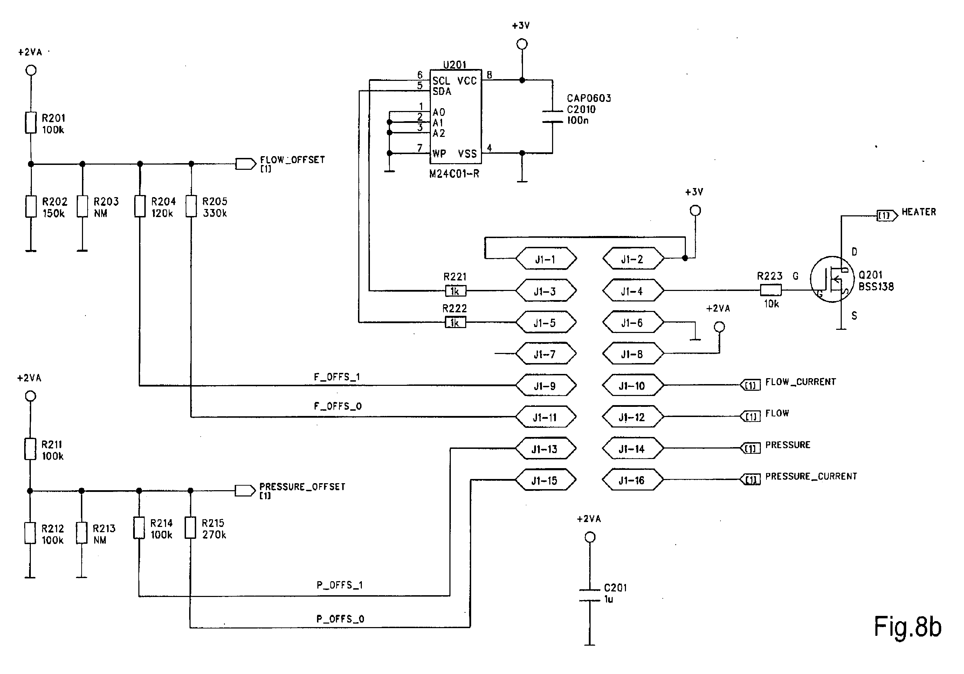

Fig. 8b is a circuit diagram showing miscellaneous components of the sensor circuit board. A drain pin of a power transistor Q201 is connected to the heating resistor Rh, and a gate pin of the same transistor Q201 is connected to and controlled from the microcontroller U101.

A calibration memory circuit comprising a memory circuit U201 is connected to the microcontroller U101

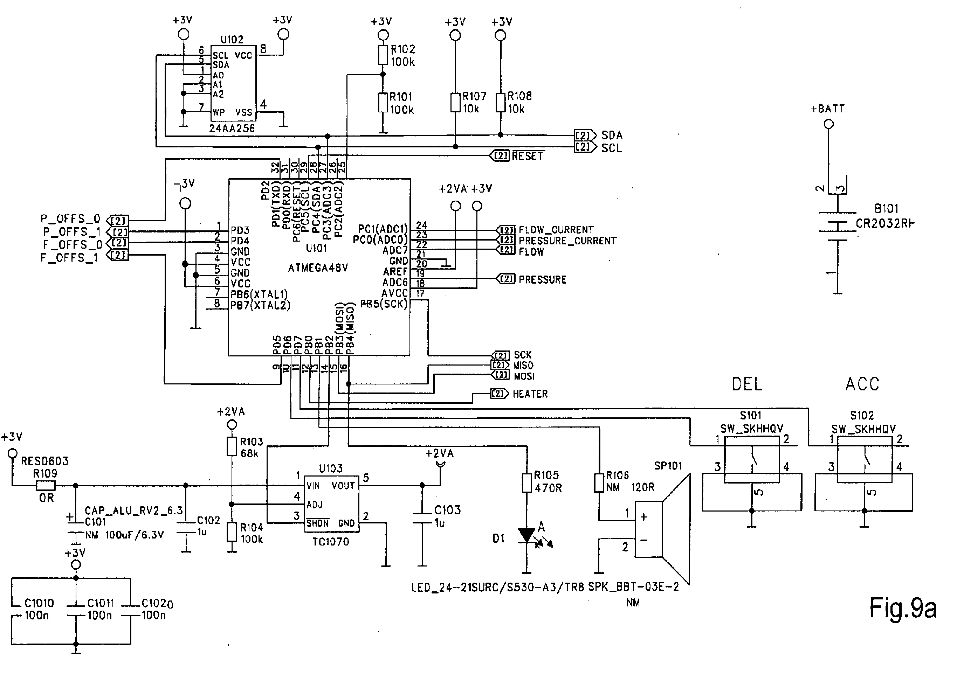

Fig. 9a shows a circuit diagram of a controller circuit board for a drainage device. The microcontroller U101 mentioned above is also connected to two control button switches S101 ,denoted "DEL" and S 102 denoted "ACC" making it possible for the operator to instruct the microcontroller to initiate different functions, such as e.g. displaying the accumulated flow during the last 1, 3 or 6 hours. The microcontroller is also connected to a light emitting diode D1 and a speaker SP101, and a display organ (not shown)

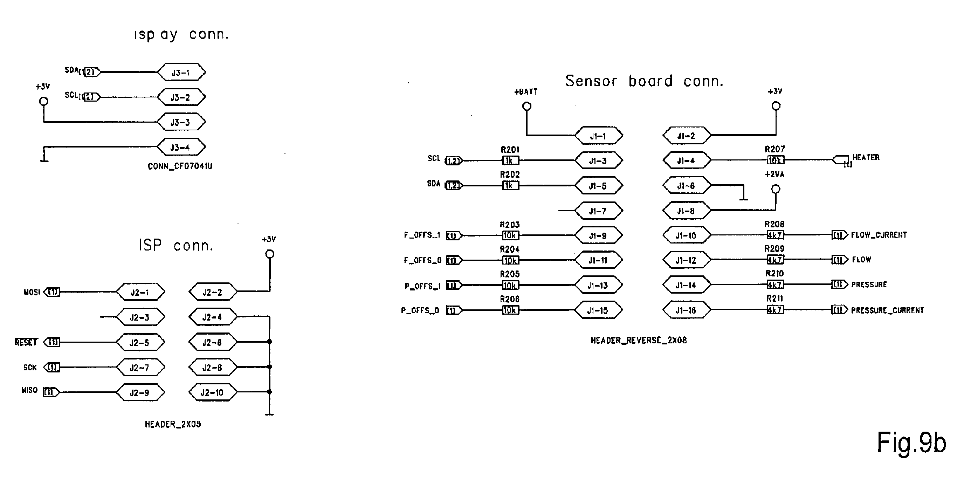

Fig. 9b.shows a connectors overview.

Other air flow sensors useable in a drainage device are listed below.

### 1. Thermistor

A very small thermistor is heated and its resistance measured. For lowest cost, the thermistor will be unheated/heated in periods to also measure the ambient temperature.

### 2. Vortex

A laminar flow is disturbed with a small edge, and the turbulence caused is analyzed. Said turbulence is analyzed according to parameters such as the sound frequency and sound volume which is created and that corresponds to the air speed. Such sound parameters is measured with a small microphone and processed by a processor.

### 3. Moving vane

A flexible vane is introduced in the air stream, and said vanes deflection is measured. Measuring can be accomplished by an optic measuring device.

### 4. Hot wire

A very thin wire is heated with constant power or held at constant temperature. The power or the temperature is measured.

### 5. Rotating vane

A propeller covers the entire air duct and its rotation is measured.

### 6. Pitot tube

A pitot tube is connected to a pressure sensor and the pressure corresponds to the square root of the air speed

### 7. Differential pressure

Pressure is measured at two locations, and the difference between the points shall correspond to the air speed

### 8. Doppler

Sound is sent through the moving air, and the time taken for the sound to travel from the transmitter to a receiver is affected by the air speed.

### 9. Cavity resonance

A tuned cavity is introduced in the air stream, and the volume of the resonant tone is measured

### Software controlled functionality

Airflow and suction pressure measurement functions of a system according to an embodiment of the present invention can be implemented in software.

### Airflow measurement processing

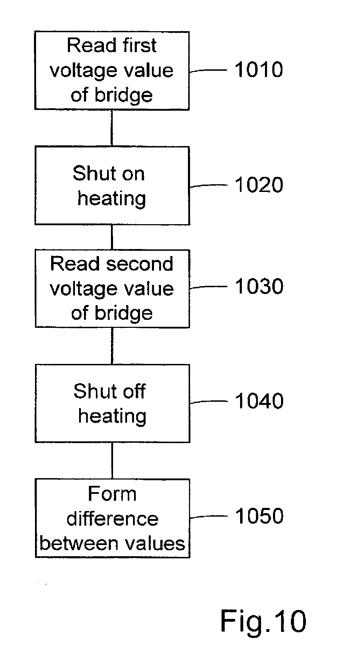

Fig. 10 shows a flow chart of a method of obtaining a value representative of an air flow for use with one embodiment of the invention.

A first voltage corresponding to a first difference in temperature between a first and a second resistor in a MEMS airflow sensor device as described above is read 1010 into the memory of a microcontroller unit. Subsequently, the microcontroller unit initiates heating 1020 of the sensor hearing resistor. After a short period, a second voltage corresponding to a second difference in temperature between said first and second resistors, is read 1030 into the memory of the microcontroller unit. The microcontroller unit subsequently shut off heating 1040. The microcontroller unit then calculates a difference 1050 between said first and second voltage value. Said difference is representative of the flow passing the sensor and is translated to a flow value by means of a translation function which can be implemented as a table. The procedure is then repeated.

### Pressure

A voltage value representative of the suction pressure in the drainage device is read into the memory of the microcontroller unit. A current value representative of the temperature of the pressure sensor is read into the memory of the microcontroller unit.

### Signal processing

The electronics provide sensor signal conditioning and compensation, data storage and data display. The electronics is battery powered and is intended for one-time use. An LCD display is used to present measurement data. The electronics is partitioned into two boards because of environmental considerations. The sensors need to be disposed as medical waste, while it is desirable that as large as possible portion of the electronics can be recycled. This is especially important for the battery. The boards are denoted sensor board and controller board. For the case when using the product as a single use model each sensor board will have a unique serial number. Once a controller board has been connected to a sensor board it shall not accept any other sensor board. This is accomplicshed by software functionality reading identification numbers and then storing said numbers. The next time one of the boards is connected to the same, or another corresponding board these numbers will be checked, and if a mismatch occurs, the boards will be set in an error state.

Signal processing is performed in the microcontroller unit. The drainage device is adapted to measure, store and display pressure variations and air leakage during the breathing cycle for example postoperatively or the like. The outputs include a display of intrathoracic pressure during breathing, and also a presentation of the present air leakage. The data is saved and could later be retrieved bedside or via a computer.

### Display of collected data

The micro-controller is programmed such that when an operator presses the ACC button once, the accumulated value of the last hour will be displayed on the LCD. One horizontal line will be highlighted on the leftmost position on the LCD to indicate the chosen time range. When pressing the ACC button twice within two seconds, the mean value of the accumulated values of the last 3 hours will be displayed on the LCD. Two horizontal lines on the leftmost position on the LCD will be highlighted. When pressing the ACC button three times within two seconds, the mean value of the accumulated values of the last 6 hours will be displayed on the LCD. Three horizontal lines on the leftmost position on the LCD will be highlighted

The micro-controller is programmed such that when the operator presses the DEL button for 5 seconds the pressure and flow data stored in the memory, but not the sensor/controller ID, will be erased.

The data memory is placed on the controller board. The controller board is adapted to be detachable and it is devised to be able to be put in a reader connected to a computer such that said computer can retrieve the data. The controller board is provided with a connector through which the contents of the data memory can be read via a special reader. This will allow receiving the total data collected and displaying it as a graph function or the like.

### Display pressure segment

Preferably, display pressure segments (analogue scale) show zero at the six o'clock position. Positive pressure values are presented clockwise and negative pressures counterclockwise. Zero pressure is represented by no segments highlighted.

### Alarm management

Preferably, an alarm LED D 1 is adapted to flash when pressure is outside the preferably -40 mbar to +5 mbar region. When pressure is back in the region, the LED D1 is turned off.

## Claims

1. An air flow sensor (Fig.7a, 7b, 107) useable in a drainage apparatus (199) for collecting fluid from a body cavity of a patient by suction, wherein said sensor is adapted to sense the cooling effect of an airflow passing a number of measurement resistors (R1, R2, R3, R4) and as an output provide a signal representative of a value of said airflow, and where the resistors are physically arranged on a chip such that when in use the air flow to be measured is flowing over and cooling the resistors, the sensor comprising four measurement resistors (R1, R2, R3, R4) physically arranged along a reference line parallel to a flow direction of the flow which flow rate is to be measured and where said four measurement resistors comprise a first resistor (R1) being arranged leftmost with reference to said line, a second resistor (R2) being arranged to the right of said first resistor with reference to said line, a third resistor (R3) being arranged to the right of said second resistor with reference to said line and a forth resistor (R4) being arranged to the right of said third resistor with reference to said line, the sensor comprising an electrical bridge circuit comprising said resistors and a first, a second, a third, and a fourth connection point (P1-P4), and wherein
said third resistor (R3) is connected between the first (P1) and the second (P2) connection points, the second resistor (R2) is connected between the second (P2) and the third (P3) connection points, and the fourth resistor (R4) is connected between the third (P3) and fourth (P4) connection points, and the first resistor (R1) is connected between the fourth (P4) and the first (P1) connection points,wherein said bridge is fed with a voltage over the first (P1) and the third (P3) connection points, and a voltage Vout representative of said flow is measured over the second (P2) and fourth (P4) connection points, and wherein a heating resistor (Rh) is physically arranged between the second (R2) and the third (R3) resistors with reference to the reference line, the sensor further comprising means for alternately energising and not energising the heating resistor (Rh), and also comprising means to form a difference value Vdiff between a first voltage value Vout1 measured when the heating resistor is in a non heated state, and a second voltage value Vout2 measured when the heating resistor is in a heated state,
**characterised in that** the difference value Vdiff is further used as being representative of the flow and being relatively insensitive to offset, the sensor also comprising means for compensating the flow measurement for offset caused by temperature shift, and wherein said means for compensating makes use of the fact that the current flowing through a bridge circuit exposed to the air which temperature is to be compensated for, is representative of said temperature, the sensor also comprising a pressure sensor bridge circuit, and a value representative of the current through said pressure sensor bridge is used to compensate the flow sensor for temperature variations.

## Patentansprüche

1. Einen Luftströmungssensor (Fig. 7a, 7b, 107), welcher in einer Drainagevorrichtung (199) zum Sammeln einer Flüssigkeit von einer Körperkavität eines Patienten durch Absaugen verwendbar ist, wobei der Sensor dazu geeignet ist, den Kühleffekt eines Luftstromes zu messen, welcher eine Anzahl von Messwiderständen (R1, R2, R3, R4) umströmt, und als Ausgabe ein Signal bereitzustellen, das repräsentativ für einen Wert dieses Luftstromes ist, und wo die Widerstände physisch derart auf einem Chip angeordnet sind, dass im Gebrauch der zu messende Luftstrom über die Widerstände strömt und diese dabei kühlt, wobei der Sensor vier Messwiderstände (R1, R2, R3, R4) aufweist, welche physisch entlang einer Referenzlinie parallel zu einer Strömungsrichtung der Strömung, deren Strömungsrate zu messen ist, angeordnet sind, und wo diese vier Messwiderstände einen in Bezug auf diese Linie ganz links angeordneten, ersten Widerstand (R1) aufweisen, einen zweiten Widerstand (R2), welcher in Bezug auf diese Linie rechts vom ersten Widerstand angeordnet ist, einen dritten Widerstand (R3), welcher in Bezug auf diese Linie rechts vom zweiten Widerstand angeordnet ist und einen vierten Widerstand (R4) aufweisen, welcher in Bezug auf diese Linie rechts vom dritten Widerstand angeordnet ist, wobei der Sensor eine elektrische Brückenschaltung aufweist, welche diese Widerstände und einen ersten, einen zweiten, einen dritten und einen vierten Verbindungspunkt (P1-P4) aufweist, und wobei der dritte Widerstand (R3) zwischen den ersten (P1) und den zweiten (P2) Verbindungspunkten verbunden ist, der zweite Widerstand (R2) zwischen den zweiten (P2) und den dritten (P3) Verbindungspunkten verbunden ist, und der vierte Widerstand (R4) zwischen den dritten (P3) und vierten (P4) Verbindungspunkten verbunden ist, und der erste Widerstand (R1) zwischen den vierten (P4) und den ersten (P1) Verbindungspunkten verbunden ist, wobei die Brücke mit einer Spannung über die ersten (P1) und die dritten (P3) Verbindungspunkte gespiesen ist, und eine Spannung Vout, welche repräsentativ für diese Strömung ist, über die zweiten (P2) und vierten (P4) Verbindungspunkte gemessen wird, und wobei ein Heizwiderstand (Rh) in Bezug auf die Referenzlinie physisch zwischen den zweiten (R2) und den dritten (R3) Widerständen angeordnet ist, wobei der Sensor ausserdem Mittel aufweist, um wechselweise den Heizwiderstand (Rh) zu aktivieren und zu deaktivieren, und zudem Mittel aufweist, um einen Differenzwert Vdiff zu bilden zwischen einem ersten Spannungswert Vout1, der gemessen wird, wenn der Heizwiderstand in einem nicht beheizten Zustand ist, und einem zweiten Spannungswert Vout2, der gemessen wird, wenn der Heizwiderstand in einem beheizten Zustand ist, **dadurch gekennzeichnet, dass** der Differenzwert Vdiff zudem dadurch verwendet wird, dass er repräsentativ ist für die Strömung und verhältnismässig unempfindlich ist gegenüber einem Offset, wobei der Sensor auch ein Mittel aufweist, um die Strömungsmessung in Bezug auf einen Offset, der durch Temperaturveränderung verursacht ist, zu kompensieren, und wobei dieses Mittel zum Kompensieren von der Tatsache Gebrauch macht, dass der Strom, welcher durch eine Brückenschaltung fliesst, die der Luft, deren Temperatur zu kompensieren ist, ausgesetzt ist, repräsentativ für diese Temperatur ist, wobei der Sensor zudem eine Druckmessungs-Brückenschaltung aufweist, und wobei ein Wert, welcher repäsentativ für den Strom durch diese Druckmessungs-Brücke ist, verwendet wird, um den Strömungssensor in Bezug auf Temperaturschwankungen zu kompensieren.

## Revendications

1. Capteur d'écoulement d'air (Figure 7a, 7b, 107) utilisable dans un dispositif de drainage (199) pour collecter un fluide à partir d'une cavité corporelle d'un patient par aspiration, dans lequel ledit capteur est adapté pour détecter l'effet de refroidissement d'un écoulement d'air qui passe à travers un certain nombre de résistances de mesure (R1, R2, R3, R4), et pour générer en sortie un signal représentatif d'une valeur dudit écoulement d'air, et dans lequel les résistances sont physiquement agencées sur une puce de telle sorte que lorsqu'elles sont utilisées, l'écoulement d'air à mesurer s'écoule sur et refroidisse les résistances, le capteur comprenant quatre résistances de mesure (R1, R2, R3, R4) qui sont agencées physiquement le long d'une ligne de référence qui est parallèle à une direction d'écoulement de l'écoulement dont le débit doit être mesuré, et dans lequel lesdites quatre résistances de mesure comprennent une première résistance (R1), qui est disposée à l'extrême gauche par rapport à ladite ligne, une deuxième résistance (R2) qui est disposée à droite de ladite première résistance par rapport à ladite ligne, une troisième résistance (R3) qui est disposée à droite de ladite deuxième résistance par rapport à ladite ligne, et une quatrième résistance (R4) qui est disposée à droite de ladite troisième résistance par rapport à ladite ligne, le capteur comprend un circuit de pont électrique comprenant lesdites résistances et des premier, deuxième, troisième et quatrième points de connexion (P1-P4), et dans lequel ladite troisième résistance (R3) est connectée entre le premier (P1) et le deuxième (P2) points de connexion, la deuxième résistance (R2) est connectée entre le deuxième (P2) et le troisième (P3) points de connexion, la quatrième résistance (R4) est connectée entre le troisième (P3) et le quatrième (P4) points de connexion, et la première résistance (R1) est connectée entre le quatrième (P4) et le premier (P1) points de connexion, dans lequel ledit pont est alimenté avec un voltage sur les premier (P1) et troisième (P3) points de connexion, et un voltage Vₒᵤₜ représentative dudit écoulement est mesurée sur le deuxième (P2) et le quatrième (P4) points de connexion, et dans lequel une résistance chauffante (Rh) est physiquement agencée entre la deuxième (R2) et la troisième (R3) résistances par rapport à la ligne de référence, le capteur comprenant en outre des moyens pour exciter et désexciter de façon alternative la résistance chauffante (Rh), et comprenant en outre des moyens pour former une valeur de différence V_{diff} entre une première valeur de voltage Vₒᵤₜ₁ mesurée lorsque la résistance chauffante se trouve dans un état non chauffé, et une deuxième valeur de voltage Vₒᵤₜ₂ mesurée lorsque la résistance chauffante se trouve dans un état chauffé, **caractérisé en ce que** la valeur de différence V_{diff} est en outre utilisée comme étant représentative de l'écoulement et étant relativement insensible à un offset, le capteur comprenant en outre des moyens pour compenser la mesure de l'écoulement pour l'offset provoqué par le changement de température, et dans lequel lesdits moyens de compensation exploitent le fait que le courant qui s'écoule à travers un circuit de pont exposé à l'air dont la température doit être compensée est représentatif de ladite température, le capteur comprenant en outre un circuit de pont de capteurs de pression, et une valeur représentative du courant qui traverse ledit pont de capteurs de pression est utilisée pour compenser le capteur d'écoulement pour des variations de température.
